# EUROPÄISCHE PATENTSCHRIFT

(11) **EP 3 630 714 B1**
(45) Veröffentlichungstag und Bekanntmachung des Hinweises auf die Patenterteilung: **16.06.2021**
(21) Anmeldenummer: 18722001.7
(22) Anmeldetag: 26.04.2018
(51) Int. Cl.: C07C 49/08, C07C 45/82, B01D 3/00, B01D 3/14

(54) **VERFAHREN UND VORRICHTUNG ZUR AUFREINIGUNG VON ACETON/WASSERGEMISCHEN MITTELS DOPPEL EFFEKT DESTILLATION**
METHOD AND APPARATUS FOR PURIFYING ACETONE/WATER MIXTURES USING DOUBLE EFFECT DISTILLATION
PROCÉDÉ ET DISPOSITIF DE NETTOYAGE DE MÉLANGE ACÉTONE/EAU À L'AIDE D'UNE DISTILLATION À DOUBLE EFFET

(30) Priorität: 31.05.2017 EP 17173746
(43) Veröffentlichungstag der Anmeldung: 08.04.2020
(73) Patentinhaber: Rhodia Acetow GmbH, 79108 Freiburg (DE)
(72) Erfinder: HUMMEL, Andreas, 79108 Freiburg (DE); KRUMREY, Thomas, 79331 Teningen (DE)
(74) Vertreter: Trinks, Ole
(86) Internationale Anmeldenummer: PCT/EP2018/060734
(87) Internationale Veröffentlichungsnummer: WO 2018/219560

(56) Entgegenhaltungen:
- CN-U- 201 701 768
- FR-A1- 2 549 043
- YOU XINQIANG ET AL: "Reducing process cost and CO2emissions for extractive distillation by double-effect heat integration and mechanical heat pump", APPLIED ENERGY, ELSEVIER SCIENCE PUBLISHERS, GB, Bd. 166, 28. Januar 2016 (2016-01-28), Seiten 128-140, XP029471194, ISSN: 0306-2619, DOI: 10.1016/J.APENERGY.2016.01.028
- YOUNG HAN KIM: "Energy saving and thermodynamic efficiency of a double-effect distillation column using internal heat integration", KOREAN JOURNAL OF CHEMICAL ENGINEERING, SPRINGER US, BOSTON, Bd. 29, Nr. 12, 17. August 2012 (2012-08-17), Seiten 1680-1687, XP035150489, ISSN: 1975-7220, DOI: 10.1007/S11814-012-0106-0
- DIAZ, V.H.G. ET AL.: "Techno-economic analysis of extraction-based separation systems for acetone, butanol, and ethanol recovery and purification", BIORESOURCES AND BIOPROCESSING, Bd. 4, Nr. 12, 13. Februar 2017 (2017-02-13), Seiten 1-13, XP002773538,

## Beschreibung

Die vorliegende Erfindung betrifft ein Verfahren zur Abtrennung von Aceton aus Aceton/Wassergemischen unter Nutzung der DED-Technik (Double Effect Destillation), bei der zwei Rektifikationskolonnen bei unterschiedlichem Druck gefahren werden, so dass die Abwärme des Kopfprodukts einer unter Druck gefahrenen Kolonne zur Erwärmung des Sumpfprodukts einer bei niedrigerem Druck gefahrenen Kolonne genutzt werden kann. Die vorliegende Erfindung betrifft weiterhin eine Vorrichtung zur Durchführung eines entsprechenden Verfahrens.

Zum Zweck der Energieeinsparung bei Destillationsanlagen ist die sogenannte DED-Technik (Double Effect Distillation) seit Langem bekannt. Bei dieser Technik werden zwei (oder mehrere) Anlagen bei verschiedenen Drücken gefahren, wodurch sich verschiedene Siedetemperaturen im Kopf und Sumpf der jeweiligen Kolonnen ergeben. Durch geeignete Abstimmung der Drücke kann eine Wärmekopplung zwischen diesen Kolonnen aufgebaut werden, so dass das Kopfprodukt einer bei höherem Druck betriebenen Kolonne den Sumpf einer bei niedrigerem Druck gefahrenen Kolonne beheizen kann. Durch diese Maßnahme lässt sich entsprechend Heizenergie einsparen.

Bei der Acetonrückgewinnung stellt sich hierbei allerdings das Problem, dass die Siedetemperaturen von Kopf und Sumpfprodukt insbesondere bei Aceton/Wassergemischen relativ weit auseinander liegen (55°C zu 102°C bei Normal-druck). Dies hat zur Folge, dass eine "Vakuumkolonne" in einer DED-Anlage so weit im Vakuum betrieben werden muss, dass die Sumpftemperatur mindestens 5 Kelvin unter der Kopftemperatur von 55°C einer Normaldruckkolonne liegt. Dies wird bei etwa 80 mbar absolut erreicht, ist aber mit dem erheblichen Nachteil verbunden, dass aufgrund des niedrigen Vakuums die erforderlichen Kolonnen sehr groß dimensioniert werden müssen, was mit hohen Investitionskosten verbunden ist. Zudem erfolgt die Kondensation am Kopf bei diesen Vakuum-Kolonnen bei etwa 0°C, so dass die Verwendung von preisgünstigen Kühlmedien, wie z.B. Kühlturmwasser bzw. Luftkühlung, nicht mehr möglich ist. Eine Wärmerückgewinnung mit dem Zulauf ist ebenfalls nicht mehr möglich.

Eine alternative Variante einer Double Effect Distillation besteht in der Kopplung einer Normaldruckkolonne mit einer Druckkolonne. Hierbei muss der Druck auf mindestens 5 bar absolut angehoben werden, damit eine Wärmekopplung, wie oben beschrieben, möglich wird. Bei diesem Drücken bildet sich jedoch ein Azeotrop von Wasser und Aceton, so dass die Reinheit des über die Druckkolonne erhaltenen Acetons signifikant geringer ist als die Reinheit des über die Normaldruckkolonne erhaltenen Acetons. Darüber hinaus steigt bei höheren Temperaturen das Risiko der Bildung von Abbauprodukten des Acetons, wie insbesondere von Diacetonalkohol und Mesityloxid.

Bisher wurde eine solche Verfahrensführung z.B. in der FR 2549043 für die Aufreinigung von wässrigen Lösungen von Butanol und Aceton vorgeschlagen, wobei in einer ersten Stufe die organischen Bestandteile Butanol und Aceton auf einen Gehalt von etwa 60% angereichert werden, während in einer zweiten Stufe Aceton und Butanol vollständig voneinander getrennt werden. Die FR 2549043 gibt an, dass die erste Destillationsstufe mit zwei Destillationssäulen ausgestaltet sein kann, wobei eine erste Destillationssäule bei erhöhtem Druck von 3 bis 10 bar absolut und eine zweite Destillationssäule bei einem Druck von 0,5 bis 1,5 bar absolut gefahren wird. Da dieses Verfahren in der ersten Stufe jedoch kein hinlänglich reines Aceton als Endprodukt liefert (der Wassergehalt nach der ersten Stufe beträgt etwa 40%; reines Aceton wird erst in der zweiten Destillationsstufe generiert), ist das Verfahren der FR 2549043 mit dem Nachteil verbunden, dass eine weitere Destillationsstufe erforderlich ist, um die erforderliche Reinheit zu erreichen.

Eine Kombination beider Alternativen könnte einen Kompromiss darstellen, wobei eine Kolonne mit einem leichten Vakuum mit einer Druckkolonne, die bei leichtem Überdruck gefahren wird, kombiniert wird.

Bei bestehenden Installationen mit einer bereits vorhandenen Normaldruckkolonne ist diese Kombination jedoch mit erheblich höheren Investitionskosten verbunden als die Kombination einer Normaldruckkolonne mit einer Druckkolonne bzw. einer Niederdruckkolonne und einer Normaldruckkolonne.

Vor dem Hintergrund dieses Standes der Technik bestand ein Bedarf für die Bereitstellung eines energieökonomischen Verfahrens zur Aufreinigung und Abtrennung von Aceton aus Aceton/Wassergemischen, bei dem die vorstehend geschilderten Nachteile weitestgehend vermieden werden und ein möglichst geringer spezifischer Energiebedarf realisiert werden kann. Die vorstehende Erfindung befasst sich mit diesem Bedarf.

Gemäß einem ersten Aspekt betrifft die vorstehende Erfindung demzufolge ein Verfahren zur Abtrennung von Aceton aus Aceton/Wassergemischen, umfassend
- Aufkonzentrieren eines Teilstroms des Aceton/Wassergemisches in mindestens einer Kolonne, die bei Überdruck betrieben wird, wobei ein Produkt mit einer Acetonkonzentration von mindestens 80 Gew.-% erhalten wird,
- Aufkonzentrieren eines Teilstroms des Aceton/Wassergemisches in mindestens einer Kolonne, die bei Normaldruck betrieben wird,
wobei das Kopfprodukt aus der Kolonne, die bei Überdruck betrieben wird, über einen Wärmetauscher zum Erwärmen des Sumpfprodukts der Kolonne, die unter Normaldruck betrieben wird, genutzt wird, und wobei das Kopfprodukt anschließend oberhalb der Zufuhr des Teilstroms des Aceton/Wassergemisches in die Kolonne, die unter Normaldruck betrieben wird, eingeleitet wird.

Demzufolge entspricht das erfindungsgemäße Verfahren im Wesentlichen einem regulären Destillationsverfahren unter Nutzung der Double Effect-Destillationstechnik, bei dem in Kauf genommen wird, dass sich im Rahmen der Aufkonzentration eines Teilstroms des Aceton/Wassergemisches in einer Kolonne, die bei Überdruck betrieben wird, keine optimale Trennung von Aceton und Wasser realisieren lässt. Dieser Nachteil wird jedoch dadurch kompensiert, dass das zumindest in Bezug auf Aceton wesentlich angereicherte Aceton/Wassergemisch in die Aufkonzentrierungskolonne, die bei Normaldruck betrieben wird, eingeleitet wird, so dass dort eine vollständige Trennung ermöglicht wird. Diese Prozessführung hat insbesondere bei in Bezug auf Aceton niedrig konzentrierten Aceton/Wassergemischen den Vorteil, dass nur eine geringe Menge eines schon relativ stark aufkonzentrierten Acetons als Kopfprodukt der unter Druck betriebenen Kolonne in die unter Normaldruck betriebene Kolonne eingeleitet wird. Über den Wärmetauscher wird die in der Druckkolonne eingesetzte Energie weitestgehend oder im Wesentlichen vollständig wiederverwertet. Die Zuführung des in Bezug auf Aceton angereicherten Aceton/Wassergemisches bewirkt zudem, dass das Rücklaufverhältnis in der bei Normaldruck betriebenen Kolonne stark reduziert werden kann, wodurch sich der Gesamtenergiebedarf der kombinierten Anlage weiter reduziert. Von Vorteil ist dabei auch, dass sich die hydraulische Belastung des Kolonnen-teils, in den das Acetongemisch zugeführt wird nur unwesentlich ändert.

Wenn im Vorstehenden von "oberhalb" oder "unterhalb" in Bezug auf Kolonnen die Rede ist, so bedeutet "oberhalb" in diesem Zusammenhang, dass "oben" in Bezug auf die Kolonne den Teil der Kolonne, der bei niedrigerer Temperatur betrieben wird, und "unten" den Teil der Kolonne, der bei höherer Temperatur betrieben wird, bezeichnet. Demzufolge wird das Sumpfprodukt im unteren Bereich der Kolonne und das Kopfprodukt im oberen Bereich der Kolonne erhalten.

Unter "Normaldruck" ist im Kontext der vorliegenden Erfindung ein Druck im Bereich von 0,5 bis 1,5 und bevorzugt 0,8 bis 1,2 bar absolut zu verstehen.

Die Kolonne, die bei Überdruck betrieben wird, wird im Rahmen der vorliegenden Erfindung in der Regel bei einem Druck im Bereich von 4 bis 11 bar absolut betrieben, wobei jedoch ein Überdruck im Bereich von 4,5 bis 8 bar absolut und insbesondere von 5 bis 7 bar absolut als bevorzugt angegeben werden kann. Als am meisten bevorzugt gilt im Rahmen der vorliegenden Erfindung für die bei Überdruck betriebene Kolonne ein Druck von etwa 6 bar absolut.

Unter "Aceton/Wassergemisch" ist im Kontext der vorliegenden Erfindung ein Gemisch, welches aus Aceton und Wasser besteht, zu verstehen; desweiteren ist darunter ebenfalls ein Gemisch zu verstehen, das im Wesentlichen aus Aceton und Wasser besteht. "Im Wesentlichen" bedeutet hier ein Gemisch, das zu mindestens 95% Gew.% aus Aceton und Wasser, bevorzugt zu 97 bis 99,9 Gew.-% aus Wasser und Aceton besteht. Weitere Inhaltsstoffe, die in dem Wasser/Acetongemisch mit bis zu 5 Gew.% enthalten sein können, sind, z.B. Kohlenwasserstoffe, beispielsweise sog. Spinnöl, das bei der Herstellung von Celluloseacetatfasern eingesetzt wird, Diacetonalkohol oder Mesityloxid. Das Aceton/Wassergemisch, aus welchem in dem Verfahren der vorliegenden Erfindung Aceton abgetrennt wird, stammt bevorzugt aus einem Spinnverfahren zur Herstellung von Celluloseacetatfasern, wie es beispielsweise in P. Rustemeyer, Macromol. Symp. 2004, 208, 267-291 beschrieben wird.

Im Rahmen der vorliegenden Erfindung ist es weiterhin zweckmäßig, wenn in der Kolonne, die unter Überdruck betrieben wird, ein Kopfprodukt erhalten wird, das eine Acetonkonzentration von mindestens 90 Gew.-%, bevorzugt 90 bis 99,9 Gew.-%, stärker bevorzugt 93 bis 98 Gew.-% und weiter bevorzugt 94 Gew.-% bis 96 Gew.-%, aufweist.

Im Rahmen des geschilderten Verfahrens ist es zweckmäßig, wenn die Abwärme aus dem Sumpfprodukt der bei Überdruck betriebenen Kolonne für das Vorerwärmen von in den Prozess zugeführtem Aceton/Wassergemisch genutzt wird. Besonders zweckmäßig ist es, wenn das aus der Kolonne, die bei Überdruck betrieben wird, erhaltene Sumpfprodukt zur Vorerwärmung des in die Kolonne eingebrachten Aceton/Wassergemisches genutzt wird. Eine entsprechende Nutzung der Abwärme lässt sich mit Hilfe eines Wärmetauschers, in dem die Abwärme aus dem Sumpfprodukt an das der Kolonne zugeführte Aceton/Wassergemisch abgegeben werden kann, realisieren.

Im Rahmen des vorstehend geschilderten Verfahrens sind die bei Überdruck und Normaldruck betriebenen Kolonnen zweckmäßig so auszugestalten, dass eine ausreichende Trennung von Aceton und Wasser gewährleistet ist.

Für die Kolonne, die bei Überdruck betrieben wird, ist es hierbei bevorzugt, wenn sie 30 bis 60 und bevorzugt 40 bis 50 theoretische Böden aufweist.

Für die Kolonne, die bei Normaldruck betrieben wird, gilt eine Anzahl von 20 bis 40 und bevorzugt 25 bis 35 theoretischen Böden als geeignet.

Die Temperatur im Sumpfprodukt der Kolonne, die bei Überdruck betrieben wird, beträgt im Rahmen des hier angegebenen Verfahrens vorzugsweise 130 bis 180°C, insbesondere 150 bis 170°C und meist bevorzugt etwa 160°C. Die Temperatur des Kopfprodukts dieser Kolonne beträgt zweckmäßig etwa 100 bis 130°C und insbesondere etwa 105 bis 120°C. Um eine ausreichend effiziente Wärmeübertragung an das Sumpfprodukt der Kolonne, die bei Normaldruck betrieben wird, zu gewährleisten, sollte die Temperatur des Kopfprodukts zudem bevorzugt mindestens 5°C und insbesondere mindestens 10°C höher sein als die Temperatur des Sumpfprodukts in der Kolonne, die bei Normaldruck betrieben wird.

Das Verfahren ist erfindungsgemäß zweckmäßig so auszugestalten, dass das Aceton in der mindestens einen Kolonne, die bei Normaldruck betrieben wird, auf eine Konzentration von mindestens 98 Gew.-% und besonders bevorzugt mindestens 98,5 Gew.-%, stärker bevorzugt mindestens 98,5 bis 99,9 Gew.-% aufkonzentriert wird. Alternativ oder zusätzlich dazu ist es zweckmäßig, wenn die Kolonne, die bei Normaldruck betrieben wird, mit einem Rücklaufverhältnis im Bereich von 2 bis 5 und bevorzugt von 3 bis 4 betrieben wird.

Das im Rahmen des erfindungsgemäßen Verfahrens aus der Kolonne, die bei Normaldruck betrieben wird, erhaltene Kopfprodukt wird zweckmäßig in einem Kühler kondensiert, um flüssiges Aceton zu erhalten. Hierzu wird bevorzugt reguläres Kühlwasser verwendet.

Durch die Auslegung des erfindungsgemäßen Verfahrens, bei der eine Wärmekopplung der mindestens einen Kolonne, die bei Überdruck betrieben wird und der mindestens einen Kolonne, die bei Normaldruck betrieben wird, erfolgt, wird eine erhebliche Energieeinsparung möglich.

Der spezifische Energieverbrauch des erfindungsgemäßen Verfahrens lässt sich noch weiter senken, wenn eine dritte Kolonne vorgesehen wird, die unter Vakuum betrieben wird. In dieser Ausführungsform der Erfindung ist das erfindungsgemäße Verfahren daher so ausgestaltet, dass es neben den oben genannten Schritten des Weiteren umfasst:
- Aufkonzentrieren eines Teilstroms des Aceton/Wassergemisches in mindestens einer Kolonne, die unter Vakuum betrieben wird,
wobei das Kopfprodukt aus der Kolonne, die bei Normaldruck betrieben wird, über einen Wärmetauscher zum Erwärmen des Sumpfproduktes der Kolonne, die unter Vakuum betrieben wird, genutzt wird.

Unter "Vakuum" ist in Kontext der vorliegenden Erfindung ein Druck im Bereich von <0,5, insbesondere 0,05 bis <0,5 bar absolut zu verstehen.

Diese Ausführungsform der Erfindung führt durch das Vorsehen einer dritten Kolonne zu einer Erhöhung der Investitionskosten, senkt jedoch im Gegenzug die Energiekosten für den laufenden Betrieb noch weiter.

Zusätzlich kann in dieser Ausführungsform der Erfindung, bei der mindestens drei Kolonnen vorgesehen sind, das Verfahren so ausgelegt werden, dass das Sumpfprodukt aus der Kolonne, die bei Normaldruck betrieben wird, unterhalb der Zufuhr des Teilstroms des Aceton/Wassergemisches in die Kolonne, die unter Vakuum betrieben wird, eingeleitet werden kann.

Diese Ausführungsform hat den zusätzlichen Vorteil, dass eine noch stärkere Abreicherung von Aceton im Sumpfprodukt der Kolonne, die bei Normaldruck betrieben wird, erreicht wird. Dies ist unter umweltschutztechnischen Gesichtspunkten und damit verbunden auch unter wirtschaftlichen Gesichtspunkten vorteilhaft.

Ein weiterer Aspekt der vorliegenden Erfindung betrifft eine Vorrichtung zur Durchführung eines Verfahrens, wie es vorstehend beschrieben wurde, wobei die Vorrichtung Folgendes umfasst:
- mindestens eine Kolonne 1 zur Auftrennung eines Teilstroms eines Aceton/ Wassergemisches, die für einen Betrieb mit Überdruck ausgelegt ist,
- mindestens eine Kolonne 2 zur Auftrennung eines Teilstroms eines Aceton/ Wassergemisches, die für einen Betrieb mit Normaldruck ausgelegt ist,
- Zuleitungen für Teilströme 7, 8 eines Aceton/Wassergemisches zu den Trennkolonnen,
- mindestens einen Wärmetauscher 3, der in fließender Verbindung mit dem Kopf der Kolonne 1, die für einen Betrieb mit Überdruck ausgelegt ist, und mit dem Boden der Kolonne 2, die für einen Betrieb mit Normaldruck ausgelegt ist, steht, sodass ein Wärmeaustausch des Kopfprodukts der Kolonne 1, die für einen Betrieb mit Überdruck ausgelegt ist, und des Sumpfprodukts der Kolonne 2, die für einen Betrieb mit Normaldruck ausgelegt ist, ermöglicht wird,
- eine Zuleitung 5 für das Kopfprodukt der Kolonne 1, die für einen Betrieb mit Überdruck ausgelegt ist, mit der der Wärmetauscher 3 mit dem oberen Teil der Kolonne 2, die für einen Betrieb mit Normaldruck ausgelegt ist, verbunden ist,
wobei die Zuleitung 5 oberhalb der Zuleitung 7 von einem Teilstrom eines Aceton/Wassergemisches zur Kolonne 2, die für einen Betrieb mit Normal-druck ausgelegt ist, an der Kolonne 2 angebracht ist.

In einer Ausführungsform ist die Vorrichtung so ausgestaltet, dass im Bereich der Zuleitung 5 eine Auffangvorrichtung für das Kopfprodukt vorgesehen ist, in der dieses vor der Zuleitung in die Kolonne 2 auf den dort vorhandenen Druck entspannt wird. Die Auffangvorrichtung, z.B. in Form eines Kessel oder äquivalenten Reservoirs, ist in dieser Ausführungsform im Bereich der Zuleitung 5 zwischen dem Wärmetauscher 3 und der Kolonne 2 positioniert.

Darüber hinaus ist es für die geschilderte Vorrichtung bevorzugt, wenn sie eine Kondensationsvorrichtung für in der Kolonne, die für einen Betrieb mit Normal-druck ausgelegt ist, generiertes Kopfprodukt aufweist.

Schließlich ist es für die erfindungsgemäße Vorrichtung bevorzugt, dass sie einen Wärmetauscher 10 aufweist, der in fließender Verbindung mit dem Sumpfprodukt der Kolonne 1, die für einen Betrieb mit Überdruck ausgelegt ist, und mit der Zuleitung 8 für den Teilstrom eines Aceton/Wassergemisches zu dieser Kolonne steht.

Im Folgenden soll die vorliegende Erfindung unter Verweis auf die Figuren näher erläutert werden.

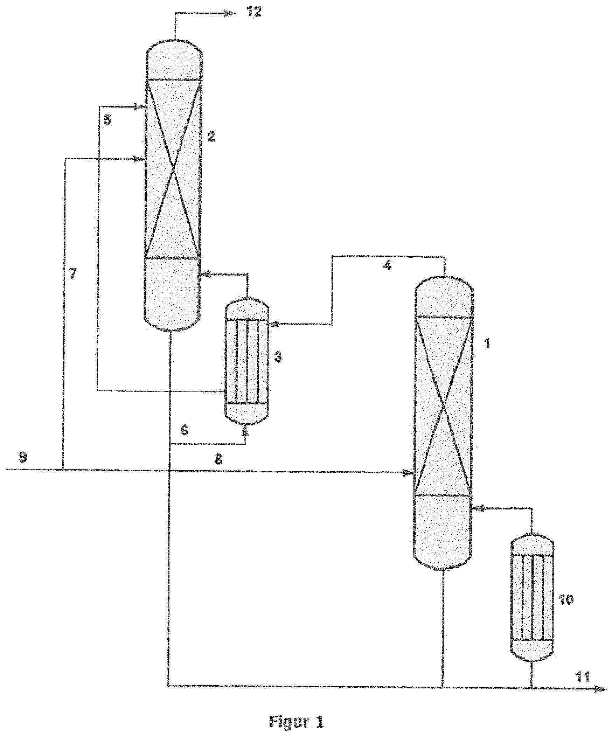

Figur 1 zeigt eine erfindungsgemäße Vorrichtung, bei der eine Kolonne 1, die für einen Betrieb mit Überdruck ausgelegt ist, und eine Kolonne 2, die für einen Betrieb mit Normaldruck ausgelegt ist, miteinander verschaltet sind. Das Kopfprodukt, das vorzugsweise eine Acetonkonzentration von mindestens 90 Gew.-%, bevorzugt 90 bis 99,9 Gew.-%, stärker bevorzugt 93 Gew.-% bis 98 Gew.-%, und weiter bevorzugt 94 Gew.-% bis 96 Gew.-% aufweist, wird über die Ableitung 4 in einen Wärmetauscher 3 überführt und von dort über die Zuleitung 5 in die Kolonne 2 eingeleitet. Aus einer gemeinsamen Zuleitung 9 wird über die Teilleitungen 7 und 8 Aceton/Wassergemisch in die Kolonnen 1 und 2 geleitet. Der Wärmetauscher 3 ist ebenfalls über den Kreislauf 6 mit dem Sumpfprodukt der Kolonne 2 verbunden, um über die Abgabe aus Abwärme aus dem Kopfprodukt der Kolonne 1 an das Sumpfprodukt der Kolonne 2 einen Wärmeaustausch zu ermöglichen. Aus der Kolonne 2 wird als Kopfprodukt im Wesentlichen reines Aceton über die Ableitung 12 erhalten. Unter im Wesentlichen reinem Aceton wird im Sinne der vorliegenden Anmeldung vorzugsweise ein Produkt verstanden, das einen Acetongehalt von mindestens 98 Gew.-%, vorzugsweise mindestens 98,5 Gew.-%, insbesondere 98,5 bis 99,9 Gew.-% aufweist. Das in der Trennkolonne 1 generierte Sumpfprodukt ist ebenfalls in einem Kreislauf mit einem Wärmetauscher 10 eingebunden, indem dem Sumpfprodukt zusätzliche Wärme beispielsweise über entsprechend hoch erhitzten Dampf zugeführt werden kann. Überschüssiges Sumpfprodukt wird als Abwasser über die Ableitung 11 aus der erfindungsgemäßen Vorrichtung freigesetzt. Die Abwasserleitung 11 ist zudem mit dem Sumpfproduktkreislauf 6 der Kolonne 2 verbunden, um dort anfallendes überschüssiges Sumpfprodukt abzuführen.

Nach angestellten Berechnungen kann durch die Verfahrensführung und Vorrichtung gemäß der vorliegenden Erfindung eine signifikante Reduktion des spezifischen Energiebedarfs um etwa 35 bis 45% erzielt werden. Gegenüber den weiter oben beschriebenen theoretischen Alternativen verringert sich das Investitionsvolumen ebenfalls erheblich, da nur eine neue Kolonne benötigt wird und ggf. vorhandene Normaldruckkolonnen ohne größere Änderung eingebunden werden können.

In einer Ausführungsform der erfindungsgemäßen Vorrichtung ist eine dritte Kolonne vorgesehen, die für den Betrieb unter Vakuum ausgelegt wird.
In dieser Ausführungsform umfasst die erfindungsgemäße Vorrichtung folgendes:
- mindestens eine Kolonne 1 zur Auftrennung eines Teilstroms eines Aceton/ Wassergemisches, die für einen Betrieb mit Überdruck ausgelegt ist,
- mindestens eine Kolonne 2a zur Auftrennung eines Teilstroms eines Aceton/Wassergemisches, die für einen Betrieb mit Normaldruck ausgelegt ist,
- mindestens eine Kolonne 2b zur Auftrennung eines Teilstroms eines Aceton/Wassergemisches, die für einen Betrieb unter Vakuum ausgelegt ist,
- Zuleitungen für Teilströme 7a, 7b, 8 eines Aceton/Wassergemisches zu den Trennkolonnen,
- mindestens einen Wärmetauscher 3a, der in fließender Verbindung mit dem Kopf der Kolonne 1, die für einen Betrieb mit Überdruck ausgelegt ist, und mit dem Boden der Kolonne 2a, die für einen Betrieb mit Normaldruck ausgelegt ist, steht, sodass ein Wärmeaustausch des Kopfprodukts der Kolonne 1, die für einen Betrieb mit Überdruck ausgelegt ist, und des Sumpfprodukts der Kolonne 2a, die für einen Betrieb mit Normaldruck ausgelegt ist, ermöglicht wird,
- mindestens einen Wärmetauscher 3b, der in fließender Verbindung mit dem Kopf der Kolonne 2a, die für einen Betrieb mit Normaldruck ausgelegt ist, und mit dem Boden der Kolonne 2b, die für einen Betrieb unter Vakuum ausgelegt ist, steht, sodass ein Wärmeaustausch des Kopfprodukts der der Kolonne 2a, die für einen Betrieb mit Normaldruck ausgelegt ist, und des Sumpfprodukts der Kolonne 2b, die für einen Betrieb unter Vakuum ausgelegt ist, ermöglicht wird,
- eine Zuleitung 5 für das Kopfprodukt der Kolonne 1, die für einen Betrieb mit Überdruck ausgelegt ist, mit der der Wärmetauscher 3a mit dem oberen Teil der Kolonne 2a, die für einen Betrieb mit Normaldruck ausgelegt ist, verbunden ist,
wobei die Zuleitung 5 oberhalb der Zuleitung 7a von einem Teilstrom eines Aceton/Wassergemisches zur Kolonne 2a, die für einen Betrieb mit Normaldruck ausgelegt ist, an der Kolonne 2a angebracht ist.

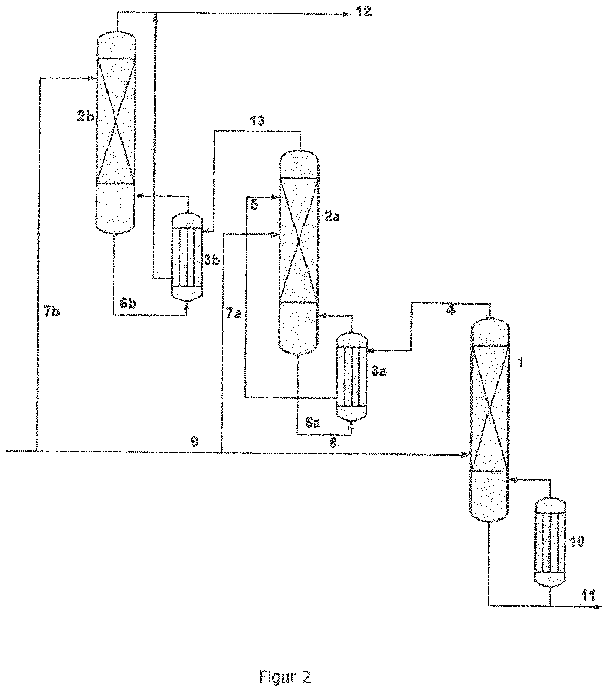

Diese Ausführungsform der erfindungsgemäßen Vorrichtung ist in Fig. 2 gezeigt.

Bei dieser Ausführungsform der Erfindung kann zusätzlich eine weitere Zuleitung vorgesehen sein, mit der das Sumpfprodukt der Kolonne 2a, die mit Normaldruck betrieben wird unterhalb der Zufuhr des Teilstroms des Aceton/Wassergemisches in die Kolonne 2b, die unter Vakuum betrieben wird, eingeleitet werden kann. Dieses zusätzliche Merkmal ermöglicht eine noch stärkere Abreicherung von Aceton im Sumpfprodukt der Kolonne 2a.

### Bezugszeichenliste

- 1: Kolonne für Betrieb bei Überdruck
- 2, 2a, 2b: Kolonne für Betrieb bei Normaldruck
- 3, 3a, 3b: Wärmetauscher
- 4, 4a, 4b: Zuleitung von Kopfprodukt zu Wärmetauscher
- 5: Zuleitung von Kopfprodukt in Kolonne 2
- 6, 6a, 6b: Sumpfproduktkreislauf
- 7, 7a, 7b: Zuleitung für Teilstrom zu Kolonne 2
- 8: Zuleitung für Teilstrom zu Kolonne 1
- 9: Hauptleitung für Aceton/Wassergemisch
- 10: Wärmetauscher
- 11: Ableitung Abwasser
- 12: Ableitung Aceton
- 13: Tank
- 14a, 14b: Zuleitung von Kopfprodukt in Kolonnen 2a und 2b
- 15: Rückführleitung zu Kolonne 1
- 16: Kondensationsvorrichtung
- 17: Kondensationsvorrichtung

## Patentansprüche

1. Verfahren zur Abtrennung von Aceton aus Aceton/Wassergemischen, umfassend
- Aufkonzentrieren eines Teilstroms des Aceton/Wassergemisches in mindestens einer Kolonne, die bei Überdruck betrieben wird, wobei ein Produkt mit einer Acetonkonzentration von mindestens 80 Gew.-% erhalten wird,
- Aufkonzentrieren eines Teilstroms des Aceton/Wassergemisches in mindestens einer Kolonne, die bei Normaldruck betrieben wird,
wobei das Kopfprodukt aus der Kolonne, die bei Überdruck betrieben wird, über einen Wärmetauscher zum Erwärmen des Sumpfprodukts der Kolonne, die unter Normaldruck betrieben wird, genutzt wird, und wobei das Kopfprodukt anschließend oberhalb der Zufuhr des Teilstroms des Aceton/ Wassergemisches in die Kolonne, die unter Normaldruck betrieben wird, eingeleitet wird.

2. Verfahren gemäß Anspruch 1, **dadurch gekennzeichnet, dass** die Kolonne, die bei Überdruck betrieben wird, bei einem Überdruck im Bereich von 4,5 bis 8 bar absolut, bevorzugt 5 bis 7 bar absolut und meist bevorzugt etwa 6 bar absolut betrieben wird.

3. Verfahren gemäß Anspruch 1 oder 2,
**dadurch gekennzeichnet, dass**
in der Kolonne, die unter Überdruck betrieben wird, ein Kopfprodukt mit einer Acetonkonzentration von mindestens 90 Gew.-%, bevorzugt 90 bis 99,9 Gew.-%, stärker bevorzugt 93 Gew.-% bis 98 Gew.-%, und weiter bevorzugt 94 Gew.-% bis 96 Gew.-% erhalten wird.

4. Verfahren gemäß irgendeinem der vorstehenden Ansprüche,
**dadurch gekennzeichnet, dass**
das aus der Kolonne, die bei Überdruck betrieben wird, erhaltene Sumpfprodukt zur Vorwärmung des in die Kolonne eingebrachten Aceton/Wassergemisches genutzt wird.

5. Verfahren gemäß irgendeinem der vorstehenden Ansprüche,
**dadurch gekennzeichnet, dass**
die Kolonne, die bei Überdruck betrieben wird, 30 - 60, bevorzugt 40 - 50 theoretische Böden aufweist.

6. Verfahren gemäß irgendeinem der vorstehenden Ansprüche,
**dadurch gekennzeichnet, dass**
die Kolonne, die bei Normaldruck betrieben wird, 20 - 40, bevorzugt 25 -35 theoretische Böden aufweist.

7. Verfahren gemäß irgendeinem der vorstehenden Ansprüche,
**dadurch gekennzeichnet, dass**
das Verfahren des Weiteren umfasst:
- Aufkonzentrieren eines Teilstroms des Aceton/Wassergemisches in mindestens einer Kolonne, die unter Vakuum betrieben wird,
wobei das Kopfprodukt aus der Kolonne, die bei Normaldruck betrieben wird, über einen Wärmetauscher zum Erwärmen des Sumpfproduktes der Kolonne, die unter Vakuum betrieben wird, genutzt wird.

8. Verfahren gemäß Anspruch 7,
**dadurch gekennzeichnet, dass**
das Sumpfprodukt aus der Kolonne, die bei Normaldruck betrieben wird, unterhalb der Zufuhr des Teilstroms des Aceton/Wassergemisches in die Kolonne, die unter Vakuum betrieben wird, eingeleitet wird.

9. Verfahren gemäß irgendeinem der vorstehenden Ansprüche,
**dadurch gekennzeichnet, dass**
das Aceton in der mindestens einen Kolonne, die bei Normaldruck betrieben wird, auf eine Konzentration von mindestens 98% Gew.-%, bevorzugt mindestens 98,5 Gew.-%, stärker bevorzugt mindestens 98,5 bis 99,9 Gew.-% aufkonzentriert wird.

10. Verfahren gemäß irgendeinem der vorstehenden Ansprüche,
**dadurch gekennzeichnet, dass**
die Kolonne, die bei Normaldruck betrieben wird, mit einem Rücklaufverhältnis von 1 bis 2 betrieben wird.

11. Vorrichtung zur Durchführung eines Verfahrens gemäß einem der Ansprüche 1 bis 10, umfassend:
- mindestens eine Kolonne (1) zur Auftrennung eines Teilstroms eines Aceton/Wassergemisches, die für einen Betrieb mit Überdruck ausgelegt ist,
- mindestens eine Kolonne (2) zur Auftrennung eines Teilstroms eines Aceton/Wassergemisches, die für einen Betrieb mit Normaldruck ausgelegt ist,
- Zuleitungen für Teilströme (7,8) eines Aceton/Wassergemisches zu den Trennkolonnen,
- mindestens einen Wärmetauscher (3), der in fließender Verbindung mit dem Kopf der Kolonne (1), die für einen Betrieb mit Überdruck ausgelegt ist, und mit dem Boden der Kolonne (2), die für einen Betrieb mit Normaldruck ausgelegt ist, steht, sodass ein Wärmeaustausch des Kopfprodukts der Kolonne (1), die für einen Betrieb mit Überdruck ausgelegt ist, und des Sumpfprodukts der Kolonne (2), die für einen Betrieb mit Normaldruck ausgelegt ist, ermöglicht wird,
- eine Zuleitung (5) für das Kopfprodukt der Kolonne (1), die für einen Betrieb mit Überdruck ausgelegt ist, mit der der Wärmetauscher (3) mit dem oberen Teil der Kolonne (2), die für einen Betrieb mit Normaldruck ausgelegt ist, verbunden ist, wobei
die Zuleitung (5) oberhalb der Zuleitung (7) für einen Teilstrom eines Aceton/Wassergemisches mit der Kolonne, die für einen Betrieb mit Normaldruck ausgelegt ist, verbunden ist.

12. Vorrichtung nach Anspruch 11,
**dadurch gekennzeichnet, dass**
sie eine Kondensationsvorrichtung für in der Kolonne (2), die für einen Betrieb mit Normaldruck ausgelegt ist, generiertes Kopfprodukt aufweist.

13. Vorrichtung nach mindestens einem der Ansprüche 11 oder 12,
**dadurch gekennzeichnet, dass**
sie einen Wärmetauscher (10) aufweist, der in fließender Verbindung mit dem Sumpfprodukt der Kolonne, die für einen Betrieb mit Überdruck ausgelegt ist, und mit der Zuleitung (8) für den Teilstrom eines Aceton/Wassergemisches zu dieser Kolonne steht.

14. Vorrichtung nach mindestens einem der Ansprüche 11 bis 13, **dadurch gekennzeichnet, dass** die Vorrichtung umfasst:
- mindestens eine Kolonne (1) zur Auftrennung eines Teilstroms eines Aceton/ Wassergemisches, die für einen Betrieb mit Überdruck ausgelegt ist,
- mindestens eine Kolonne (2a) zur Auftrennung eines Teilstroms eines Aceton/Wassergemisches, die für einen Betrieb mit Normaldruck ausgelegt ist,
- mindestens eine Kolonne (2b) zur Auftrennung eines Teilstroms eines Aceton/Wassergemisches, die für einen Betrieb unter Vakuum ausgelegt ist,
- Zuleitungen für Teilströme (7a, 7b, 8) eines Aceton/Wassergemisches zu den Trennkolonnen,
- mindestens einen Wärmetauscher (3a), der in fließender Verbindung mit dem Kopf der Kolonne (1), die für einen Betrieb mit Überdruck ausgelegt ist, und mit dem Boden der Kolonne (2a), die für einen Betrieb mit Normaldruck ausgelegt ist, steht, sodass ein Wärmeaustausch des Kopfprodukts der Kolonne (1), die für einen Betrieb mit Überdruck ausgelegt ist, und des Sumpfprodukts der Kolonne (2a), die für einen Betrieb mit Normaldruck ausgelegt ist, ermöglicht wird,
- mindestens einen Wärmetauscher (3b), der in fließender Verbindung mit dem Kopf der Kolonne (2a), die für einen Betrieb mit Normaldruck ausgelegt ist, und mit dem Boden der Kolonne (2b), die für einen Betrieb unter Vakuum ausgelegt ist, steht, sodass ein Wärmeaustausch des Kopfprodukts der Kolonne (2a), die für einen Betrieb mit Normaldruck ausgelegt ist, und des Sumpfprodukts der Kolonne (2b), die für einen Betrieb unter Vakuum ausgelegt ist, ermöglicht wird,
- eine Zuleitung (5) für das Kopfprodukt der Kolonne (1), die für einen Betrieb mit Überdruck ausgelegt ist, mit der der Wärmetauscher (3a) mit dem oberen Teil der Kolonne (2a), die für einen Betrieb mit Normaldruck ausgelegt ist, verbunden ist,
wobei die Zuleitung (5) oberhalb der Zuleitung (7a) für einen Teilstrom eines Aceton/Wassergemisches zur Kolonne (2a), die für einen Betrieb mit Normaldruck ausgelegt ist, verbunden ist.

## Claims

1. A method for separating acetone from acetone/water mixtures, comprising:
- concentrating a partial flow of the acetone/water mixture in at least one column operated under positive pressure, wherein a product having an acetone concentration of at least 80 wt% is obtained,
- concentrating a partial flow of the acetone/water mixture in at least one column operated under normal pressure,
wherein the top product from the column operated under positive pressure is used to heat the bottom product of the column operated under normal pressure via a heat exchanger, and wherein the top product is subsequently introduced into the column operated under normal pressure above the supply of the partial flow of the acetone/ water mixture.

2. The method according to claim 1, **characterized in that** the column operated under positive pressure is operated at a positive pressure in the range of 4.5 to 8 bar absolute, preferentially 5 to 7 bar absolute, and most preferably at approximately 6 bar absolute.

3. The method according to claim 1 or 2,
**characterized in that**
a top product having an acetone concentration of at least 90 wt%, preferentially 90 to 99.9 wt%, more preferably 93 to 98 wt%, and further preferably 94 to 96 wt%. is obtained in the column operated under positive pressure.

4. The method according to any one of the preceding claims, **characterized in that**
the bottom product obtained from the column operated under positive pressure is used to preheat the acetone/water mixture introduced into the column.

5. The method according to any one of the preceding claims, **characterized in that**
the column operated under positive pressure exhibits 30 - 60, preferentially 40 - 50 theoretical plates.

6. The method according to any one of the preceding claims, **characterized in that**
the column operated under normal pressure exhibits 20 - 40, preferentially 25 - 35 theoretical plates.

7. The method according to any one of the preceding claims, **characterized in that**
the method further comprises:
- concentrating a partial flow of the acetone/water mixture in at least one column operated under a vacuum,
wherein the top product from the column operated under normal pressure is used to heat the bottom product of the column operated under vacuum via a heat exchanger.

8. The method according to claim 7,
**characterized in that**
the bottom product from the column operated under normal pressure is introduced into the column operated under vacuum below the supply of the partial flow of the acetone/water mixture.

9. The method according to any one of the preceding claims, **characterized in that**
the acetone in the at least one column operated under normal pressure is concentrated to a concentration of at least 98 wt%, preferentially to at least 98.5 wt%, and further preferentially to at least 98.5 to 99.9 wt%.

10. The method according to any one of the preceding claims, **characterized in that**
the column operating under normal pressure is operated at a reflux ratio of from 1 to 2.

11. A device for realizing a method according to one of claims 1 to 10, comprising:
- at least one column (1) for separating a partial flow of an acetone/water mixture which is designed to be operated under positive pressure,
- at least one column (2) for separating a partial flow of an acetone/water mixture which is designed to be operated under normal pressure,
- supply lines for partial flows (7, 8) of an acetone/water mixture to the separation columns,
- at least one heat exchanger (3) in fluid communication with the top of the column (1) designed for operation at positive pressure and the bottom of the column (2) designed for operation at normal pressure so as to enable a transfer of heat between the top product of the column (1) designed for operation at positive pressure and the bottom product of the column (2) designed for operation at normal pressure,
- a supply line (5) for the top product of the column (1) designed for operation at positive pressure via which the heat exchanger (3) is connected to the upper part of the column (2) designed for operation at normal pressure, wherein
the supply line (5) is connected to the column designed for operation at normal pressure above the supply line (7) for a partial flow of an acetone/water mixture.

12. The device according to claim 11,
**characterized in that**
it comprises a condensation device for the top product generated in the column (2) designed to operate under normal pressure.

13. The device according to at least one of claims 11 to 12, **characterized in that**
it comprises a heat exchanger (10) in fluid communication with the bottom product of the column designed for operation at positive pressure and the supply line (8) for the partial flow of an acetone/water mixture to said column.

14. The device according to at least one of claims 11 to 13, **characterized in that** the device comprises:
- at least one column (1) for separating a partial flow of an acetone/ water mixture which is designed to be operated under positive pressure,
- at least one column (2a) for separating a partial flow of an acetone/water mixture which is designed to be operated under normal pressure,
- at least one column (2b) for separating a partial flow of an acetone/water mixture which is designed to be operated under a vacuum,
- supply lines for partial flows (7a, 7b, 8) of an acetone/water mixture to the separation columns,
- at least one heat exchanger (3a) in fluid communication with the top of the column (1) designed for operation under positive pressure and the bottom of the column (2a) designed for operation under normal pressure so as to enable a transfer of heat between the top product of the column (1) designed for operation at positive pressure and the bottom product of the column (2a) designed for operation at normal pressure,
- at least one heat exchanger (3b) in fluid communication with the top of the column (2a) designed for operation at normal pressure and the bottom of the column (2b) designed for operation under a vacuum so as to enable a transfer of heat between the top product of the column (2a) designed for operation at normal pressure and the bottom product of the column (2b) designed for operation under vacuum,
- a supply line (5) for the top product of the column (1) designed for operation at positive pressure via which the heat exchanger (3a) is connected to the upper part of the column (2a) designed for operation at normal pressure,
wherein the supply line (5) is connected to the column (2a) designed for operation at normal pressure above the supply line (7a) for a partial flow of an acetone/water mixture.

## Revendications

1. Procédé pour séparer l'acétone des mélanges acétone/eau,
consistant à
- concentrer un flux partiel du mélange acétone/eau dans au moins une colonne exploitée en surpression pour obtenir un produit ayant une concentration en acétone d'au moins 80 % en poids,
- concentrer un flux partiel du mélange acétone/eau dans au moins une colonne exploitée en pression normale,
dans lequel le produit de tête de la colonne exploitée en surpression est utilisé par un échangeur de chaleur pour chauffer le produit de fond de la colonne exploitée en pression normale, et
le produit de tête est ensuite introduit dans la colonne exploitée en pression normale, en-dessus de l'alimentation du flux partiel du mélange acétone/eau.

2. Procédé selon la revendication 1,
**caractérisé en ce que**
la colonne exploitée en surpression est exploitée à une surpression comprise entre 4,5 et 8 bars absolus, de préférence entre 5 et 7 bars absolus et plus préférablement à environ 6 bars absolus.

3. Procédé selon la revendication 1 ou 2,
**caractérisé en ce que**
dans la colonne exploitée en surpression, on obtient un produit de tête ayant une concentration en acétone d'au moins 90 % en poids, de préférence de 90 à 99,9 % en poids, plus préférablement de 93 % à 98 % en poids, et encore plus préférablement de 94 % à 96 % en poids.

4. Procédé selon l'une quelconque des revendications précédentes, **caractérisé en ce que**
le produit de fond obtenu de la colonne exploitée en surpression est utilisé pour préchauffer le mélange acétone/eau introduit dans la colonne.

5. Procédé selon l'une quelconque des revendications précédentes, **caractérisé en ce que**
la colonne exploitée en surpression comporte 30 à 60, de préférence 40 à 50 plateaux théoriques.

6. Procédé selon l'une quelconque des revendications précédentes, **caractérisé en ce que**
la colonne exploitée en pression normale comporte 20 à 40, de préférence 25 à 35 plateaux théoriques.

7. Procédé selon l'une quelconque des revendications précédentes, **caractérisé en ce que**
le procédé consiste en outre à :
- concentrer un flux partiel du mélange acétone/eau dans au moins une colonne exploitée sous vide,
dans lequel le produit de tête de la colonne exploitée en pression normale est utilisé par un échangeur de chaleur pour chauffer le produit de fond de la colonne exploitée sous vide.

8. Procédé selon la revendication 7,
**caractérisé en ce que**
le produit de fond de la colonne exploitée en pression normale est introduit dans la colonne exploitée sous vide, en-dessous de l'alimentation du flux partiel du mélange acétone/eau.

9. Procédé selon l'une quelconque des revendications précédentes, **caractérisé en ce que**
l'acétone est concentrée dans ladite au moins une colonne exploitée en pression normale jusqu'à une concentration d'au moins 98 % en poids, de préférence d'au moins 98,5 % en poids, plus préférablement d'au moins 98,5 à 99,9 % en poids.

10. Procédé selon l'une quelconque des revendications précédentes, **caractérisé en ce que**
la colonne exploitée en pression normale est exploitée à un rapport de reflux de 1 à 2.

11. Dispositif pour la mise en œuvre d'un procédé selon l'une des revendications 1 à 10, comprenant :
- au moins une colonne (1) pour séparer un flux partiel d'un mélange acétone/eau, qui est conçue pour être exploitée en surpression,
- au moins une colonne (2) pour séparer un flux partiel d'un mélange acétone/eau, qui est conçue pour être exploitée en pression normale,
- des conduites d'alimentation pour des flux partiels (7, 8) d'un mélange acétone/eau vers les colonnes de séparation,
- au moins un échangeur de chaleur (3) qui est en communication fluidique avec la tête de la colonne (1) conçue pour être exploitée en surpression et avec le plateau de la colonne (2) conçue pour être exploitée en pression normale, de manière à permettre un échange de chaleur du produit de tête de la colonne (1) conçue pour être exploitée en surpression, et du produit de fond de la colonne (2) conçue pour être exploitée en pression normale,
- une conduite d'alimentation (5) pour le produit de tête de la colonne (1) conçue pour être exploitée en surpression, par laquelle l'échangeur de chaleur (3) est relié à la partie supérieure de la colonne (2) conçue pour être exploitée en pression normale,
dans lequel
la conduite d'alimentation (5) est reliée à la colonne conçue pour être exploitée en pression normale, en-dessus de la conduite d'alimentation (7) pour un flux partiel d'un mélange acétone/eau.

12. Dispositif selon la revendication 11,
**caractérisé en ce que**
il comprend un dispositif de condensation du produit de tête généré dans la colonne (2) conçue pour être exploitée en pression normale.

13. Dispositif selon l'une au moins des revendications 11 ou 12, **caractérisé en ce que**
il comprend un échangeur de chaleur (10) en communication fluidique avec le produit de fond de la colonne conçue pour être exploitée en surpression et avec la conduite d'alimentation (8) pour le flux partiel d'un mélange acétone/eau vers ladite colonne.

14. Dispositif selon l'une au moins des revendications 11 à 13,
**caractérisé en ce que** le dispositif comprend :
- au moins une colonne (1) pour séparer un flux partiel d'un mélange acétone/eau, qui est conçue pour être exploitée en surpression,
- au moins une colonne (2a) pour séparer un flux partiel d'un mélange acétone/eau, qui est conçue pour être exploitée en pression normale,
- au moins une colonne (2b) pour séparer un flux partiel d'un mélange acétone/eau, qui est conçue pour être exploitée sous vide,
- des conduites d'alimentation pour des flux partiels (7a, 7b, 8) d'un mélange acétone/eau vers les colonnes de séparation,
- au moins un échangeur de chaleur (3a) qui est en communication fluidique avec la tête de la colonne (1) conçue pour être exploitée en surpression, et avec le plateau de la colonne (2a) conçue pour être exploitée en pression normale, de manière à permettre un échange de chaleur du produit de tête de la colonne (1) conçue pour être exploitée en surpression, et du produit de fond de la colonne (2a) conçue pour être exploitée en pression normale,
- au moins un échangeur de chaleur (3b) en communication fluidique avec la tête de la colonne (2a) conçue pour être exploitée en pression normale et avec le plateau de la colonne (2b) conçue pour être exploitée sous vide, de manière à permettre un échange de chaleur du produit de tête de la colonne (2a) conçue pour être exploitée en pression normale et du produit de fond de la colonne (2b) conçue pour être exploitée sous vide,
- une conduite d'alimentation (5) pour le produit de tête de la colonne (1) conçue pour être exploitée en surpression, par laquelle l'échangeur de chaleur (3a) est relié à la partie supérieure de la colonne (2a) conçue pour être exploitée en pression normale,
dans lequel
la conduite d'alimentation (5) est reliée à la colonne (2a) conçue pour être exploitée en pression normale, en-dessus de la conduite d'alimentation (7) pour un flux partiel d'un mélange acétone/eau.
